# EUROPÄISCHE PATENTSCHRIFT

(11) **EP 2 114 564 B1**
(45) Veröffentlichungstag und Bekanntmachung des Hinweises auf die Patenterteilung: **02.11.2016**
(21) Anmeldenummer: 07857710.3
(22) Anmeldetag: 17.12.2007
(51) Int. Cl.: B01J 23/96, B01J 23/90, B01J 21/20, B01J 21/16, B01J 23/30, B01J 23/652, B01J 23/888, B01J 29/076, B01J 35/00, C07C 45/52, B01J 37/04

(54) **VERFAHREN ZUR REGENERIERUNG EINES KATALYSATORS FÜR DIE DEHYDRATISIERUNG VON GLYCERIN**
METHOD FOR REGENERATING A CATALYST FOR DEHYDRATING GLYCERINE
PROCÉDÉ DE RÉGÉNÉRATION D'UN CATALYSEUR de DÉSHYDRATATION DE GLYCÉRINE

(30) Priorität: 29.01.2007 DE 102007004350
(43) Veröffentlichungstag der Anmeldung: 11.11.2009
(73) Patentinhaber: Evonik Degussa GmbH, 45128 Essen (DE)
(72) Erfinder: REDLINGSHÖFER, Hubert, 91481 Münchsteinach (DE); WECKBECKER, Christoph, 63584 Gründau-Lieblos (DE); HUTHMACHER, Klaus, 63571 Gelnhausen (DE); DÖRFLEIN, Andreas, 63538 Grosskrotzenburg (DE)
(86) Internationale Anmeldenummer: PCT/EP2007/064080
(87) Internationale Veröffentlichungsnummer: WO 2008/092534

(56) Entgegenhaltungen:
- EP-A- 0 598 229
- WO-A-2006/087083
- US-A- 5 608 133
- US-A1- 2006 091 045

## Beschreibung

Die Erfindung betrifft ein Verfahren zur Herstellung von Acrolein durch Dehydratisierung von Glycerin in Gegenwart eines Katalysators, der wolframhaltige Verbindungen, saure Eigenschaften und mindestens einen Promoter aufweist, und Regenerierung des verwendeten Katalysators.

Acrolein ist ein wichtiges Zwischenprodukt und von großer wirtschaftlicher Bedeutung für die Darstellung von Acrylsäure, D,L-Methionin und dem Methionin-Hydroxy-Analogen 2-Hydroxy-4-methylthiobuttersäure (MHA). Methionin ist eine essentielle Aminosäure, die u.a. als Ergänzung in Futtermitteln eingesetzt wird. Nutritivitätsverbessernde Futtermittelzusatzstoffe sind heute ein unverzichtbarer Bestandteil der Tierernährung. Sie dienen der besseren Verwertung des Nahrungsangebotes, stimulieren das Wachstum und fördern die Eiweißbildung. Einer der wichtigsten dieser Zusatzstoffe ist die essentielle Aminosäure Methionin, die vor allem in der Geflügelaufzucht als Futtermitteladditiv eine herausragende Stellung einnimmt. Auf diesem Gebiet haben, aber auch sogenannte Methionin-Ersatzstoffe wie das Methionin-Hydroxy-Analog (abgekürzt MHA) nicht unerhebliche Bedeutung, da sie ähnliche wachstumsstimulierende Eigenschaften aufweisen wie die dafür bekannte Aminosäure. Acrylsäure ist ein wichtiger Ausgangsstoff zur Herstellung von Polymeren, die beispielsweise aufgrund ihres Wasseraufnahmevermögens als Superabsorber eingesetzt werden.

Nach dem Stand der Technik erfolgt die Synthese von Acrolein durch heterogen katalysierte selektive Oxidation von Propen an Mischoxidkatalysatoren. EP 417723 beschreibt die Synthese an komplexen Multimetallmischoxidkatalysatoren bei Temperaturen von 300 bis 380 °C und Drücken von 1,4 bis 2,2 bar. In Ullmann's Encyclopedia of Industrial Chemistry, 6. Auflage, 1999 ist das gesamte Verfahren inklusive

Aufarbeitung, bei dem mehrere Nebenprodukte abgetrennt werden, beschrieben. Nachdem das Eduktgemisch aus Propen, Luft und Wasser zumindest teilweise am Katalysator umgesetzt wurde, erfolgt zunächst das Quenchen zur Abtrennung hochsiedender Nebenprodukte wie Polymere, Acrylsäure und Essigsäure. Im anschließenden Absorber wird Acrolein ausgewaschen. Nach der Desorption zur Rückgewinnung des Absorptionsmittels wird das erhaltene Rohacrolein mehrstufig destillativ gereinigt.

Es ist bekannt, dass sich Glycerin in Gegenwart saurer Stoffe zu verschiedenen Produkten dehydratisieren lässt. Gemäß Organic Synthesis I, 15-18 (1964) wird durch Behandeln eines Gemisches aus pulverförmigem Kaliumhydrogensulfat, Kaliumsulfat und Glycerin bei 190 bis 200 °C Acrolein in einer Ausbeute zwischen 33 und 48 % gewonnen. Aufgrund der niedrigen Ausbeuten und der hohen Salzfrachten eignet sich dieses Verfahren jedoch nicht für den technischen Maßstab.

Im Rahmen der Untersuchungen von Modellsubstanzen von Biomasse Pyrolyseölen wurde auch die katalytische Behandlung von Glycerin an H-ZSM5-Zeolithen bei 350 bis 500 °C untersucht - siehe Dao, Le H. et al. ACS Symp. Ser.: 376 (Pyrolysis Oils Biomass) 328-341 (1988). Kohlenwasserstoffe werden nur in geringen Ausbeuten gebildet.

In der EP 0598229, US 5387720 ist ferner die säurekatalysierte Umsetzung von Glycerin zu Acrolein in der Gas-und in der Flüssigphase beschrieben. Darin bestimmt alleine die Säurestärke (Hammet'sche Säurefunktion) die Eignung als Katalysator. Die DE 42 38 492 betrifft die Synthese von 1,2 und 1,3 Propandiol durch Dehydratisierung von Glycerin mit hohen Ausbeuten.

Festkörperkatalysatoren, die Wolframoxid auf einem Zirkonoxid- oder Titanoxidträger sowie zusätzlich ein Platingruppenmetall enthalten, sind in der US 2006/0091045 A1 beschrieben. Ferner behandelt dieses Dokument den Einsatz derartiger Katalysatoren unter anderem in der Oxidation von Dibenzothiophen zum Sulphon, der Isomerisierung von Alkanen und der Epoxidierung von Olefinen. Die Katalysatoren der US 2006/0091045 A1 lassen sich bei Temperaturen von weniger als 600°C regenerieren.

Die US 5,608,133 beschreibt ebenfalls WOₓ/ZrO₂-Katalystoren, die Hydrier- bzw. Dehydrierkomponenten als Co-Katalysator enthalten können. Die Katalysatoren der US 5,608,133 lassen sich in Anwesenheit von Wasserstoff oder Sauerstoff regenerieren.

Aus der WO 2006/087083 ist ein Verfahren zur Herstellung von Acrolein aus Glycerin an sauren Katalysatoren bekannt, bei dem man dem Reaktionsgemisch Sauerstoff zusetzt.

Ein ähnliches Verfahren wird in der WO 2006/087084 beschrieben. Die dort eingesetzten Katalysatoren weisen eine Hammett-Acidität H₀ im Bereich von -9 bis -18 auf.

Die in der Chemischen Technik eingesetzten Katalysatoren unterliegen nahezu ausnahmslos einer Desaktivierung, so dass der Katalysator in periodischen Abständen gewechselt werden muss, um eine wirtschaftliche Raum-Zeit-Ausbeute aufrechtzuerhalten. Die Standzeit der Katalysatoren ist dabei je nach Reaktionssystem sehr unterschiedlich und kann wenige Stunden bis zu viele Jahre betragen. Durch eine periodische Regenerierung des Katalysators wird dabei der Desaktivierung zumindest teilweise entgegengewirkt und die Aktivität des Katalysators wieder deutlich erhöht. Dies wird industriell häufig bei Synthesen eingesetzt, wenn sich auf dem Katalysator kohlenstoffhaltige Ablagerungen bilden, welche die aktiven Zentren bedecken. Je nach Reaktionssystem sind diese Ablagerungen unterschiedlich. Durch die erfindungsgemäße Wahl des Katalysators und den Zusatz von Promotoren, welche die Regenerierbarkeit verbessern, kann bei der Dehydratisierung von Glycerin die Raum-Zeit-Ausbeute verbessert werden.

Die Herstellung von Acrolein durch Dehydratisierung von Glycerin unter Verwendung von Festkörperkatalysatoren mit einer Hammett-Acidität von weniger als 2, die Wolframverbindungen und Palladium als Promotor enthalten, wird in der Parallelanmeldung EP 07 857 691 (Offenlegungsschrift EP 2 114 561 A1) beschrieben.

Aufgabe der Erfindung ist es, ein Verfahren zur Herstellung von Acrolein und Regenerierung des verwendeten Katalysators zur Verfügung zu stellen wobei der eingesetzte Katalysator eine geringere Verkokungsneigung zeigt und leicht regenerierbar ist.

Es wurde gefunden, dass Wolframverbindungen enthaltende Festkörperkatalysatoren mit einer Hammett-Acidität H₀ von < +2, die Palladium als Promotor enthalten, eine geringe Verkokungsneigung zeigen und leicht regenerierbar sind.

Gegenstand der Erfindung ist daher ein Verfahren zur Herstellung von Acrolein durch Dehydratisierung von Glycerin in Gegenwart von Wolframverbindungen enthaltenden Festkörperkatalysatoren mit einer Hammett-Acidität Ho von <+2, die als Promotor Palladium enthalten, wobei der Katalysator nach dem Einsatz bei der Dehydratisierung von Glycerin zu Acrolein eine geringere Aktivität und/oder Selektivität aufweist als vor diesem Einsatz und zeitlich getrennt von der Umsetzung von Glycerin periodisch regeneriert wird oder örtlich getrennt von der Umsetzung von Glycerin kontinuierlich regeneriert wird und wobei der Katalysator zur Regenerierung einer reduzierenden Atmosphäre ausgesetzt wird, ohne dass Reaktanden aus der Glycerin-Dehydratisierung anwesend sind.

Bevorzugt sind Katalysatoren, die eine Hammett-Acidität H₀ von < +2 bis -20 aufweisen.

Da Glycerin ein reaktives Molekül darstellt, das speziell bei hohen Temperaturen zur Bildung von höhersiedenden Verbindungen neigt, indem zwei oder mehrere Glycerinmoleküle miteinander reagieren, wird der Katalysator durch Ablagerungen von kohlenstoffhaltiger Molekühle auf der Oberfläche verkokt. Dies führt zur Aktivitätsverminderung.

Zur Erzielung einer hohen Raum-Zeit-Ausbeute ist nicht alleine die Säurestärke des Katalysator nach Hammett wichtig, sondern auch die Regenerierbarkeit und die Neigung zur Verkokung.

Der erfindungsgemäß eingesetzte Katalysator enthält als Promotor Palladium, das die Regeneration des Katalysators beschleunigt. Ebenfalls nehmen Standzeit und Raum-Zeit-Ausbeute deutlich zu, da vor allem die Desaktivierung durch Verkokung bei diesen Katalysatoren zumindest größtenteils beseitigt und die Aktivität deutlich erhöht wird. Somit kann der Umsatz an Glycerin und die Ausbeute zu Acrolein in Abhängigkeit von der Zeit auf hohem Niveau aufrechterhalten werden. Dies ist speziell für eine technische Umsetzung der Synthese von großer Bedeutung, da ein Wechsel des Katalysators und damit verbundene Anlagenstillstände hohe Kosten verursachen würden.

Neben den Brönsted-Säuregruppen können dabei auch Hydroxylgruppen oder Lewis-saure Zentren die Aktivität und Selektivität beeinflussen. Ebenso kann neben den Promotoren der Zusatz von Verbindungen eines oder mehrerer der Elemente zu einem Wolframverbindungen enthaltenden Katalysator, ausgewählt aus der Gruppe enthaltend Silizium, Phosphor, Niob, Zink, Zinn, Magnesium, Aluminium, Molybdän oder Vanadium oder Aktivkohle die Oberfläche des Katalysators modifizieren oder die Konzentration der aktiven Zentren verringern, so dass die Ausbeute weiter verbessert wird. Speziell die Bildung von fest adsorbierten Hochsiedern oder Koksprecursoren, die aus zwei oder mehreren benachbarten adsorbierten Glycerinmolekülen oder Zwischenverbindungen entstehen, wird dadurch verringert.

Als Festkörperkatalysatoren sind insbesondere auch die aus der US 5,387,720 (EP 0 598 229 A1) bekannten Typen geeignet, wenn sie eine Wolframverbindung und zusätzlich einen oder mehrere der genannten Promotoren enthalten. Dabei handelt es sich um feste, im Reaktionsmedium im Wesentlichen unlösliche ein- oder mehrphasig aufgebaute Stoffe mit einem Hₒ-Wert kleiner +2, vorzugsweise kleiner - 3. Der Hₒ-Wert entspricht der Säurefunktion nach Hammett und läßt sich durch die sogenannte Amintitration unter Verwendung von Indikationen oder durch Adsorption einer gasförmigen Base ermitteln - siehe Studies in surface science and catalysis, Vol. 51, 1989: "New solid acids and bases, their catalytic properties" by K. Tanabe et al. Kapitel 2, insbesondere Seiten 5-9. Kapitel 1 (Seiten 1-3) des vorgenannten Dokuments nennt zahlreiche feste Säuren, aus welchen der Fachmann, gegebenenfalls nach Bestimmung des Hₒ-Wertes, den geeigneten Katalysator für die erfindungsgemäße Modifizierung auswählen kann. Als Basis für die erfindungsgemäßen Dehydratisierungskatalysatoren eignen sich vorzugsweise (i) natürliche und synthetische silikatische Stoffe, wie insbesondere Mordenit, , saure Zeolithe und Aktivkohle; (ii) mit ein-, zwei oder mehrbasigen anorganischen Säuren oder sauren Salzen anorganischer Säuren belegte Trägerstoffe, wie oxidische oder silikatische Stoffe, beispielsweise Al₂ O₃, SiO₂, ZrO₂, TiO₂; (iii) Oxide und Mischoxide, wie beispielsweise gamma-Al₂O₃ und ZnO-Al₂O₃-, SiO₂-Al₂O₃-, ZrO₂-SiO₂, ZrO₂-HfO₂-Mischoxide oder Heteropolysäuren.

Geeignete Verbindungen zur Bereitstellung der aktiven Wolfram-Zentren können beispielsweise Ammoniumwolframat, Ammoniummetawolframat, Wolframsäure, Wolframkieselsäure, Wolframphosphorsäure oder Heteropolysäuren mit Wolfram als Bestandteil sein. Diese Verbindungen oder deren Mischungen werden dann entweder direkt als Katalysator eingesetzt oder als Katalysatorvorstufen verwendet. Bei Zusatz von weiteren Elementen erfolgt bevorzugt eine vorherige Mischung als Pulver, in einer Lösung oder in einer Schmelze, In einer Ausführungsform der Erfindung werden die katalytisch aktiven Verbindungen auf einem Träger gebunden.

Als Trägermaterialien, können beispielsweise Aluminiumoxid, Titandioxid, Siliziumdioxid, Zirkoniumdioxid, Aktivkohle oder deren Mischungen verwendet werden. Die Träger dienen vorwiegend zur Erhöhung der spezifischen Oberfläche oder zur Fixierung der aktiven Zentren.

Die Herstellung der erfindungsgemäß eingesetzten Katalysatoren erfolgt nach für den Fachmann bekannten Verfahren. Werden die aktiven Komponenten auf einen Träger aufgebracht, so erfolgt dies bevorzugt durch Imprägnieren des Trägers, wie z.B. mittels der incipient-wetness Methode durch Besprühen. Die aktiven Komponenten können auch durch Fällen oder Extraktion aus einer Lösung gewonnen werden. Anschließend kann dann eine Formgebung des Katalysators ggf. unter Zugabe von Trägern, Haftvermittlern oder Porenbildnern durch Pressen, Extrudieren, Beschichten oder Agglomerieren erfolgen. Der Katalysator weist üblicherweise einen Partikeldurchmesser zwischen 0,04 mm und 20 mm, bevorzugt zwischen 0,1 und 10 mm, insbesondere zwischen 0,5 und 7 mm auf. Die aktiven Verbindungen können auch in Form einer Schale aufgebracht sein. Wird kein Träger eingesetzt, so ist eine Katalysatorherstellung durch Extrudieren, Pressen von Tabletten oder eine Aufbauagglomeration bevorzugt.

Für die Dehydratisierung in der Gasphase werden Katalysatoren mit einem Hₒ-Wert zwischen -3 und -8,2 besonders bevorzugt; Geeignete Katalysatorsysteme, die Wolfram und als Promotor Palladium enthalten sind beispielsweise Pd/H₂WO₄, Pd/WOₓ/ZrO₂.

Die Dehydratisierung erfolgt bevorzugt in Abwesenheit von Sauerstoff. In einer Ausführungsform auch in Gegenwart von Wasserstoff in einer Menge von 0,1 bis 10 Vol.%, insbesondere von 0,5 bis 5 Vol.%, bezogen auf die Gesamtmenge des Reaktionsgemisches. Die Dehydratisierung wird in Gegenwart der oben beschriebenen Katalysatoren durchgeführt.

Die Konzentration des Glycerins im Reaktionsgemisch wird bevorzugt durch die Beimischung von unter den gewählten Reaktionsbediungen inerten geeigneten gasförmigen Verbindungen angesenkt.

Dadurch werden Nebenreaktionen zu Oligomeren, Polymeren und anderen Hochsiedern minimiert. Eingesetzt werden dem Fachmann bekannte Lösungs- und Verdünnungsmittel wie beispielsweise Wasser, Stickstoff, Luft, Kohlendioxid, Methan und/oder Wasserstoff, Alkohole wie zum Beispiel Methanol und Ethanol, Aceton, Toluol oder Methylisobutylketon. Bevorzugt werden Verdünnungsmedien, die nach der Kondensation durch Phasentrennung einfach von Acrolein isoliert werden können.

Im Reaktionsgemisch beträgt die Glycerinkonzentration zwischen 1 und 100 Gew.-%, bevorzugt zwischen 1 und 70 Gew.-% und insbesondere zwischen 5 und 40 Gew.-%

Ein Vorteil des Verfahrens besteht darin, dass auch Glycerinlösungen mit einem Gehalt von 5 bis 40 Gew.-% verwendbar sind. Somit sind sogenannte Rohglycerine ohne vorherige Aufkonzentrierung oder Reinigung direkt für die Synthese von Acrolein einsetzbar.

Die Reaktion wird bei einer Temperatur zwischen 150 und 450 °C, bevorzugt zwischen 180 und 350 °C, besonders bevorzugt zwischen 220 und 320 °C durchgeführt. Üblicherweise beträgt der Druck zwischen 0,1 und 200 bar, bevorzugt zwischen 0,5 und 50 bar, besonders bevorzugt zwischen 0,9 und 10 bar.

Das Verfahren lässt sich in der Flüssigphase oder in der Gasphase durchführen. In beiden Ausführungsformen können im Prinzip die gleichen sauren Feststoffkatalysatoren eingesetzt werden; es hat sich aber gezeigt, dass bestimmte Katalysatoren vorzugsweise für die Dehydratisierung in der Gasphase und andere, vorzugsweise für jene in der Flüssigphase, geeignet sind.

Die Umsetzung in der Gasphase wird besonders bevorzugt, weil der Glycerinumsatz praktisch vollständig ist (> 95 %)und das den Katalysator verlassende gasförmige Reaktionsgemisch unter Erhalt einer wässrigen Acroleinlösung, welche zusätzlich gebildete Nebenprodukte enthält, unmittelbar kondensiert oder absorbiert werden kann; dieses Kondensat lässt sich vielfach unmittelbar weiterverarbeiten. Die partielle Kondensation und/oder Absorption des Reaktionsgemisches kann in mehreren Stufen erfolgen. Sofern erwünscht, kann aus dem Reaktionsgemisch Acrolein, gegebenenfalls gemeinsam mit einem Teil des Wassers, durch fraktionierte Kondensation, Absorption, Desorption und abschließende Destillation gewonnen werden.

Ein Teil des Wassers wird im Kreislauf geführt, wobei es unter Nutzung der Wärmeintegration verdampft und kondensiert wird.

Auch ein Inertgas oder ein Verdünnungsmittel können im Kreislauf geführt werden.

Bei der Umsetzung in der Flüssigphase ist es zweckmäßig, die Dehydratisierung nur bis zu einem Glycerinumsatz von etwa 15 bis 25% durchzuführen, da bei einer Steigerung des Umsatzes die Selektivität abnimmt. Nach Erreichen des genannten Umsatzes wird gebildetes Acrolein allein oder zusammen mit einem Teil des Wassers in bekannter Weise, üblicherweise destillativ oder durch eine N₂-Strippung aus dem Reaktionsgemisch abgetrennt. Das Acrolein kann durch Kondensation oder eine Wäsche mit Wasser isoliert werden. Das vom Acrolein befreite glycerinhaltige Reaktionsgemisch wird in die Dehydratisierungsstufe zurückgeführt. Ein Vorteil der Dehydratisierung in der Flüssighpase gegenüber jener in der Gasphase besteht in dem geringeren Energieaufwand, weil nur das aus dem Reaktionsgemisch abgetrennte Acrolein sowie ein damit übergehender Teil Wasser verdampft werden müssen.

Die Dehydratisierung in der Gasphase erfolgt vorzugsweise im Temperaturbereich zwischen 240 und 320°C, jene in der Flüssigphase vorzugsweise zwischen 250 und 300°C. Im Falle der Flüssigphasendehydratisierung wird die Apparatur mit mindestens einem solchen Druck beaufschlagt, der zur Aufrechterhaltung der Flüssigphase erforderlich ist.

Die Dehydratisierung erfolgt in einem Festbettreaktor, einem Wirbelschichtreaktor, in einem Reaktor mit einem zirkulierenden Wirbelbett, einem Wanderbettreaktor oder einem Reaktor mit Regenerator-Riser(-Downer)-Konzept. Sie kann kontinuierlich oder diskontinuierlich durchgeführt werden.

Weiterhin ist die Kombination der Reaktion mit einer Eduktaufbereitung bzw. Produktaufarbeitung mittels einer Reaktivdestillation möglich bzw. sinnvoll, da die Siedepunktunterschiede zwischen Glycerin und Acrolein sehr groß sind. Der Katalysator ist dabei entweder im Sumpf und/oder im Kolonnenteil platziert. Der eingebrachte Katalysator kann beispielsweise in Form einer Schüttung, Suspension oder einer Beschichtung vorliegen. Ein weiterer Vorteil der Reaktivdestillation besteht darin, dass schwersiedende Verunreinigungen aus Roh-Glycerin am Sumpf der Kolonne mit weiteren Schwersiedern, die als Nebenprodukte entstehen können, ausgetragen werden. Acrolein und Leichtsieder werden dann über Kopf abgezogen.

Gebildetes Acrolein kann auch in bekannter Weise allein oder zusammen mit einem Teil des Lösungs- oder Verdünnungsmediums durch Strippen, Destillation oder Extraktion aus dem Reaktionsgemisch abgetrennt werden. Nicht umgesetztes Glycerin kann dann in die Reaktionsstufe zurückgeführt werden.

Der erfindungsgemäß eingesetzte Katalysator zeichnet sich auch durch eine gute Regenierbarkeit aus.

In Abhängigkeit von ihrem Normalpotential können einzelne Elemente nach der Regenerierung des Katalysators unter reduzierenden Bedingungen auch in metallischer Form auf dem Katalysator vorliegen.

Die Wolfram enthaltenden Verbindungen bei den Wolframverbindungen enthaltenden Festkörperkatalysatoren sind ausgewählt aus der Gruppe Ammoniumwolframat, Wolframphophorsäure, Wolframsäure, Wolframkieselsäure, Wolframoxide oder Heterophosphorsäuren mit Wolfram als Bestandteil. Besonders geeignet sind beispielsweise Pd/H₂WO₄, Pd/WOₓ/ZrO₂.

Bevorzugt enthalten die Katalysatoren natürliche oder synthetische silikatische oder oxidische Verbindungen als Träger.

Geeignet sind auch Katalysatoren, die mit ein-, zwei- oder mehrbasigen anorganischen Säuren oder Salzen anorganischer Säuren belegte Trägermaterialien enthalten.

Bevorzugt sind auch Katalysatoren, die als Trägermaterialien Aluminiumoxid, Titandioxid, Siliziumdioxid, Zirkoniumdioxid, Aktivkohle oder deren Mischungen enthalten.

Die Regeneration erfolgt bei den erfindungsgemäß eingesetzten Festkörperkatalysatoren unter Hydrierungsbedingungen. Dabei wird der Koks, der sich während der Reaktion auf der Oberfläche des Katalysators durch Ablagerung von Kohlenwasserstoffen gebildet hat, teilweise oder vollständig entfernt. Die Regeneration wird daher unter hydrierenden Bedingungen durchgeführt, da dem sauren Katalysator als Promotor Palladium mit stark hydrierender Wirkung zugesetzt wurde.

Die Regeneration erfolgt entweder zeitlich oder örtlich getrennt von der Umsetzung von Glycerin. Bei der zeitlichen Trennung wird die Einspeisung von Glycerin in den Reaktor gestoppt und daraufhin die Regeneration durchgeführt, bevor wieder das Eduktgemisch eingespeist wird. Dieser Vorgang wird dann beliebig oft periodisch wiederholt. Für die Durchführung dieser Regenerationsmethode eignet sich insbesondere die Taktung von 2 oder mehreren Festbettreaktoren, um einen kontinuierlichen Produktstrom erzeugen zu können. Dabei wird einer der Reaktoren regeneriert, während mindestens einer der Reaktoren zur Produktion von Acrolein eingesetzt wird. Die Zeitintervalle für Reaktion und Regeneration können dabei beliebig gewählt werden. Bevorzugt erfolgt die ununterbrochene Produktion von Acrolein innerhalb eines Zeitintervalls von 2 bis 3000 h, insbesondere 4 bis 400 h bevor der Katalysator in einem Zeitintervall von 0,5 bis 100 h, insbesondere 1 bis 10 h regeneriert wird.

In einer Ausführungsform des erfindungsgemäßen Verfahrens wird bei der zeitlichen Trennung des Katalysators von der Umsetzung von Glycerin die Einspeisung von Glycerin in den Reaktor gestoppt und daraufhin die Regeneration durchgeführt, bevor wieder das Eduktgemisch eingespeist wird.

Erfolgt die Regeneration örtlich getrennt, so wird der Katalysator zwischen bevorzugt 2 Reaktoren kontinuierlich bewegt. In einem der Reaktoren findet dabei kontinuierlich die Glycerinumsetzung zu Acrolein statt. Im anderen Reaktor wird der Katalysator kontinuierlich regeneriert. Geeignete Reaktorkonzepte sind dabei der Wanderbettreaktor oder das Regenerator-Riser(-Downer)-Konzept. Das Wanderbett zeichnet sich durch eine geringeren Durchsatz des Katalysators und weniger Katalysatorabrieb aus und ist hier bevorzugt.

In einer alternativen Ausführungsform des erfindungsgemäßen Verfahrens wird bei der örtlichen Trennung des Katalysators von der Umsetzung von Glycerin der Katalysator zwischen Reaktoren kontinuierlich bewegt, wobei in einem der Reaktoren kontinuierlich die Umsetzung von Glycerin zu Acrolein stattfindet und in einem anderen Reaktor der Katalysator kontinuierlich regeneriert wird.

In einer weiteren Ausführungsform des erfindungsgemäßen Verfahrens wird die Regeneration in einem Festbettreaktor, einem Wirbelschichtreaktor, in einem Reaktor mit einer zirkulierenden Wirbelschicht, einem Wanderbettreaktor oder einem Reaktor mit Regenerator-Riser(-Downer)-Konzept durchgeführt.

Zwischen der Regeneration und der Reaktion ist es jeweils sinnvoll, einen Spülschritt, bevorzugt mit Stickstoff, durchzuführen. Bei der Regeneration werden höhere Temperaturen von 100 bis 800 °C, bevorzugt 200 bis 700 °C, insbesondere 300 bis 550 °C angewandt. Diese müssen nicht mit der Reaktortemperatur während der Glycerinumsetzung übereinstimmen. Dann sind entsprechende Heiz- und Kühlschritte erforderlich. Bevorzugt wird zur Regeneration des Katalysators eine höhere Temperatur als bei der Reaktion angewandt. Der Druck bei der Regeneration beträgt bevorzugt zwischen 0 und 50 bar, insbesondere zwischen 0 und 3 bar.

In einer Ausführungsform des erfindungsgemäßen Verfahrens beträgt die Regenerationstemperatur der Wolframverbindungen enthaltenden Festkörperkatalysatoren zwischen 260 und 550°.

Zur Regeneration des Katalysators wird mindestens ein Zusatzstoff eingesetzt. Dieser ist bevorzugt gasförmig. Wird durch Hydrierung regeneriert handelt es sich um ein gasförmiges Reduktionsmittel. Bevorzugt wird dann Wasserstoff verwendet. Zur Vermeidung hoher Übertemperatur in der Katalysatorzone durch die exotherme Entfernung des Kokses wird das reduzierende Gas bevorzugt verdünnt eingesetzt, wozu beispielsweise Stickstoff oder Wasserdampf verwendet werden. Während der Regeneration des Katalysators wird die Konzentration des Zusatzstoffes bevorzugt schrittweise erhöht. Der Katalysator kann durch festes Inertmaterial verdünnt werden oder auch in verschiedenen Zonen angeordnet sein.

In einer Ausführungsform des erfindungsgemäßen Verfahrens wird als Regenerationsmittel Wasserstoff eingesetzt.

Die gewünschten katalytischen Eigenschaften und/oder die Säurefunktion des Katalysators verschwindet durch Einsatz von Wolfram und Promotoren durch die Regeneration nicht, wie beispielsweise bei den klassischen Säuren wie Phosphorsäure oder Salzsäure beobachtet wird, was wiederum zur Katalysatordesaktivierung führt.

### Beispiele

### Vergleichsbeispiel 1:

Eingesetzt wurde ein Katalysator gemäß Patentschrift DE 4238493: 100 g Siliziumoxidträger mit einem Durchmesser von etwa 4 mm wurden mit 25 g einer 20 gew.-%igen Phosphorsäure 1 h gemischt. Am Rotationsverdampfer wurde dann bei ca. 70 °C das überschüssige Wasser abgezogen. 18 ml dieses Katalysators wurden in einen Festbettreaktor mit einem Durchmesser von 15 mm gefüllt. Der Reaktor wurde dann auf eine Temperatur von 250 °C beheizt. Mittels einer Pumpe wurden 12,5 g/h einer 20 gew.-%igen wässrigen Glycerinlösung über einen auf 260 °C beheizten Verdampfer in den Reaktor geleitet. Mittels Gaschromatographie wurde der Strom am Reaktorausgang analysiert. Bis zu einer Betriebszeit von etwa 15 h konnte dabei ein vollständiger Umsatz an Glycerin beobachtet werden. Die Selektivität und somit die Ausbeute betrug dabei 79 %. Nach ca. 15 h fiel der Umsatzgrad und somit die Ausbeute jedoch steil ab, so dass nach 23 h nur noch ein Umsatzgrad von 20 % vorlag. Nachdem der Katalysator für 5 h bei einer Temperatur von 350 °C ausschließlich mit einem Wasserstoffstrom von 4 Nl/h durchströmt wurde, konnte keine Verbesserung der Ausbeute (Regenerierung) festgestellt werden. Nachdem der Katalysator für 5 h bei einer Temperatur von 350 °C ausschließlich mit einem Luftstrom von 4 Nl/h durchströmt wurde, konnte sogar eine weitere Verschlechterung der Ausbeute festgestellt werden.

### Vergleichsbeispiel 2:

Vergleichsbeispiel 1 wurde wiederholt, wobei jedoch zu Tabletten gepresste Molybdänsäure als Katalysator eingesetzt wurde. Bei einer Reaktortemperatur von 250 °C konnte innerhalb der ersten 5 h eine Ausbeute von 9 % erreicht werden. Auf eine Regenerierung wurde verzichtet.

### Vergleichsbeispiel 3:

Vergleichsbeispiel 1 wurde wiederholt, wobei jedoch zu Tabletten gepresste Wolframsäure als Katalysator eingesetzt wurde. Bei einer Reaktortemperatur von 260 °C konnte innerhalb der ersten 5 h ein vollständiger Umsatzgrad und eine Ausbeute von 79 % erreicht werden. Innerhalb der nächste Betriebsstunden verringerte sich der Umsatzgrad und entsprechend die Ausbeute deutlich. Im weiteren Verlauf war ein Abfall der Ausbeute von um ca. 5 % pro 10 h festzustellen. Nachdem der Katalysator für 10 h bei einer Temperatur von 350 °C ausschließlich mit einem Wasserstoffstrom von 4 Nl/h durchströmt wurde, konnte die Aktivität des Katalysators deutlich verbessert werden. Der Glycerinumsatz war daraufhin zu Beginn erneut vollständig. Im weiteren Verlauf verringerte sich der Umsatzgrad und die Ausbeute wie vor der Regenerierung beschrieben. Diese Taktung von Glycerindehydratisierung und Regeneration des Katalysators wurde innerhalb von 300 h drei mal wiederholt. Nach Ausbau des unregenerierten Katalysators war dieser schwarz gefärbt. Der Kohlenstoffgehalt des Katalysators betrug 22 Gew.-%, was auf eine erhebliche Verkokung hindeutet.

### Beispiel 1:

Vergleichsbeispiel 1 wurde wiederholt, wobei jedoch zu Tabletten gepresste Wolframsäure als Katalysator eingesetzt wurde. Dieser Katalysator wurde zusätzlich mit 1 Gew.-% Pd imprägniert. Dazu wurde mittels incipient-wetness Pd-Acetat eingesetzt. Bei einer Reaktortemperatur von 260 °C konnte innerhalb der ersten 5 h ein vollständiger Umsatzgrad und eine Ausbeute von 77 % erreicht werden. Innerhalb der nächste Betriebsstunden verringerte sich der Umsatzgrad und entsprechend die Ausbeute deutlich. Nachdem der Katalysator für 10 h bei einer Temperatur von 350 °C ausschließlich mit einem Wasserstoffstrom von 4 Nl/h durchströmt wurde, konnte die Aktivität des Katalysators deutlich verbessert werden.

Der Glycerinumsatz war daraufhin zu Beginn erneut vollständig. Im Vergleich zu Beispiel 1 war der Abfall des Umsatzgrades bei der Dehyratisierungsreaktion nach der Regeneration deutlich geringer und das hohe Umsatzgradniveau konnte länger eingehalten werden.

## Patentansprüche

1. Verfahren zur Herstellung von Acrolein durch Dehydratisieren von Glycerin in Gegenwart von Wolframverbindungen enthaltenden Festkörperkatalysatoren mit einer Hammett-Acidität Ho von <+2, die als Promotor Palladium enthalten, wobei der Katalysator nach dem Einsatz bei der Dehydratisierung von Glycerin zu Acrolein eine geringere Aktivität und/oder Selektivität aufweist als vor diesem Einsatz und zeitlich getrennt von der Umsetzung von Glycerin periodisch regeneriert wird oder örtlich getrennt von der Umsetzung von Glycerin kontinuierlich regeneriert wird und wobei der Katalysator zur Regenerierung einer reduzierenden Atmosphäre ausgesetzt wird, ohne dass Reaktanden aus der Glycerin-Dehydratisierung anwesend sind.

2. Verfahren gemäß Anspruch 1, **dadurch gekennzeichnet, dass** bei der zeitlichen Trennung des Katalysators von der Umsetzung von Glycerin die Einspeisung von Glycerin in den Reaktor gestoppt und daraufhin die Regeneration durchgeführt wird, bevor wieder das Eduktgemisch eingespeist wird.

3. Verfahren gemäß Anspruch 1, **dadurch gekennzeichnet, dass** bei der örtlichen Trennung des Katalysators von der Umsetzung von Glycerin der Katalysator zwischen Reaktoren kontinuierlich bewegt wird, wobei in einem der Reaktoren kontinuierlich die Umsetzung von Glycerin zu Acrolein stattfindet und im anderen Reaktor der Katalysator kontinuierlich regeneriert wird.

4. Verfahren gemäß Anspruch 1, **dadurch gekennzeichnet, dass** die Wolframverbindungen enthaltenden Festkörperkatalysatoren eine Hammett-Acidität Hₒ von < +2 bis -20 aufweisen.

5. Verfahren gemäß den Ansprüchen 1 oder 4, **dadurch gekennzeichnet, dass** bei den Wolframverbindungen enthaltenden Festkörperkatalysatoren die Wolfram enthaltenden Verbindungen ausgewählt sind aus der Gruppe Ammoniumwolframat, Wolframphosphorsäure, Wolframsäure, Wolframkieselsäure oder Heteropolysäuren mit Wolfram als Bestandteil.

6. Verfahren gemäß den Ansprüchen 1 bis 4, **dadurch gekennzeichnet, dass** die Wolframverbindungen enthaltenden Festkörperkatalysatoren als Trägermaterialien Aluminiumoxid, Titandioxid, Siliziumdioxid, Zirkoniumdioxid, Aktivkohle oder deren Mischungen enthalten.

7. Verfahren gemäß den Ansprüchen 1 bis 6, **dadurch gekennzeichnet, dass** die Regenerationstemperatur der Wolframverbindungen enthaltenden Festkörperkatalysatoren zwischen 260 und 550 °C beträgt.

8. Verfahren gemäß den Ansprüchen 1 bis 7, bei dem als Regenerations-mittel Wasserstoff eingesetzt wird.

9. Verfahren gemäß den Ansprüchen 1 bis 8, bei dem man die Regeneration in einem Festbettreaktor, einem Wirbelschichtreaktor, in einem Reaktor mit einer zirkulierenden Wirbelschicht, einem Wanderbettreaktor oder einem Reaktor mit Regenerator-Riser(-Downer)-Konzept durchführt.

## Claims

1. Process for preparing acrolein by dehydrating glycerol in the presence of solid-state catalysts which comprise tungsten compounds, have a Hammett acidity Hₒ of < +2 and contain palladium as a promoter, wherein the catalyst has a lower activity and/or selectivity after being used in the dehydration of glycerol to acrolein than prior to this use and is subjected to a periodical regeneration separated in terms of time from the conversion of glycerol or is subjected to a continuous regeneration separated in terms of place from the conversion of glycerol, and wherein the catalyst is regenerated by exposing it to a reducing atmosphere in the absence of reactants from the glycerol dehydration.

2. Process according to Claim 1, **characterized in that**, in the case of separation of the catalyst in terms of time from the conversion of glycerol, the feed of glycerol into the reactor is stopped and then the regeneration is conducted before the reactant mixture is fed in again.

3. Process according to Claim 1, **characterized in that**, in the case of separation of the catalyst in terms of place from the conversion of glycerol, the catalyst is moved continuously between reactors, with continuous conversion of glycerol to acrolein in one of the reactors and continuous regeneration of the catalyst in the other reactor.

4. Process according to Claim 1, **characterized in that** the solid-state compounds comprising tungsten compounds have a Hammett acidity Hₒ of < +2 to -20.

5. Process according to Claims 1 or 4, **characterized in that**, in the case of the solid-state catalysts comprising tungsten compounds, the tungstencontaining compounds are selected from the group of ammonium tungstate, tungstophosphoric acid, tungstic acid, tungstosilicic acid and heteropolyacids having tungsten as a constituent.

6. Process according to Claims 1 to 4, **characterized in that** the solid-state catalysts comprising tungsten compounds contain aluminium oxide, titanium dioxide, silicon dioxide, zirconium dioxide, activated carbon or mixtures thereof as support materials.

7. Process according to Claims 1 to 6, **characterized in that** the regeneration temperature of the solid-state catalysts comprising tungsten compounds is between 260 and 550°C.

8. Process according to Claims 1 to 7, in which the regenerating agent used is hydrogen.

9. Process according to Claims 1 to 8, in which the regeneration is conducted in a fixed bed reactor, a fluidized bed reactor, a reactor with a circulating fluidized bed, a moving bed reactor or a reactor with regenerator-riser(-downer) design.

## Revendications

1. Procédé pour la production d'acroléine par déshydratation de glycérol en présence de catalyseurs solides qui contiennent des composés contenant du tungstène, ayant une acidité de Hammett Ho de <+2, qui contiennent du palladium comme activateur, le catalyseur présentant après l'utilisation dans la déshydratation du glycérol en acroléine une activité et/ou une sélectivité plus faible(s) qu'avant cette utilisation et étant régénéré périodiquement séparément dans le temps d'avec la conversion du glycérol ou étant régénéré en continu séparément dans l'espace d'avec la conversion du glycérol et le catalyseur étant exposé pour la régénération à une atmosphère réductrice, sans que soient présents des partenaires réactionnels de la déshydratation du glycérol.

2. Procédé selon la revendication 1, **caractérisé en ce que** dans le cas de la séparation dans le temps du catalyseur d'avec la conversion du glycérol, on arrête l'introduction de glycérol dans le réacteur et après cela on effectue la régénération avant d'introduire de nouveau le mélange de produits de départ.

3. Procédé selon la revendication 1, **caractérisé en ce que** dans le cas de la séparation dans l'espace du catalyseur d'avec la conversion du glycérol, le catalyseur est mis en mouvement en continu entre des réacteurs, la conversion du glycérol en acroléine ayant lieu en continu dans l'un des réacteurs et le catalyseur étant régénéré en continu dans l'autre réacteur.

4. Procédé selon la revendication 1, **caractérisé en ce que** les catalyseurs solides qui contiennent des composés contenant du tungstène présentent une acidité de Hammett Hₒ de <+2 à -20.

5. Procédé selon la revendication 1 ou 4, **caractérisé en ce que** dans les catalyseurs solides qui contiennent des composés contenant du tungstène, les composés contenant du tungstène sont choisis dans le groupe constitué par le tungstate d'ammonium, l'acide phosphotungstique, l'acide tungstique, l'acide silicotungstique ou des hétéropolyacides comportant du tungstène en tant que composant.

6. Procédé selon les revendications 1 à 4, **caractérisé en ce que** les catalyseurs solides qui contiennent des composés contenant du tungstène contiennent en tant que matières de support de l'oxyde d'aluminium, du dioxyde de titane, du dioxyde de silicium, du dioxyde de zirconium, du charbon actif ou des mélanges de ceux-ci.

7. Procédé selon les revendications 1 à 6, **caractérisé en ce que** la température de régénération des catalyseurs solides qui contiennent des composés contenant du tungstène est comprise entre 260 et 550 °C.

8. Procédé selon les revendications 1 à 7, dans lequel on utilise comme agent de régénération l'hydrogène.

9. Procédé selon les revendications 1 à 8, dans lequel on effectue la régénération dans un réacteur à lit fixe, un réacteur à couche tourbillonnaire, dans un réacteur comportant une couche tourbillonnaire circulante, un réacteur à lit mobile ou un réacteur à concept Regenerator-Riser(-Downer).
